Europäisches Patentamt

European Patent Office

Office européen des brevets

(19)

(11) Publication number: **0 132 359**
**A2**

(12) # EUROPEAN PATENT APPLICATION

(21) Application number: **84304842.2**

(22) Date of filing: **16.07.84**

(51) Int. Cl.⁴: **C 07 K 15/12,** C 12 P 21/02
// C12N5/00, A61K37/02

(30) Priority: **19.07.83 PC /JP83/002 31**

(43) Date of publication of application: **30.01.85**
**Bulletin 85/5**

(84) Designated Contracting States: **AT BE CH DE FR GB IT**
**LI LU NL SE**

(71) Applicant: **Takeda Chemical Industries, Ltd., 27,**
**Doshomachi 2-chome Higashi-ku, Osaka-shi**
**Osaka, 541 (JP)**

(72) Inventor: **Sugino, Hiromu 458-6, Kamigoryonaka-machi,**
**Muromachi-dori, Kuramaguchi-sagaru Higashi-iru,**
**Kamigyo-ku Kyoto-shi Kyoto 602 (JP)**
Inventor: **Kawahara, Kenji, 428-57, Fuseya-cho, Izumi-shi**
**Osaka 594 (JP)**
Inventor: **Kato, Kouichi, 2-49, Fushimidai 1-chome**
**Inagawa-cho, Kawabe-gun Hyogo 666-02 (JP)**
Inventor: **Tsukamoto, Kyozo,**
**A-204, 39 Senriyamanishi 4-chome, Suita-shi**
**Osaka 565 (JP)**

(74) Representative: **Laredo, Jack Joseph, Elkington and Fife**
**High Holborn House 52/54 High Holborn, London,**
**WC1V 6SH (GB)**

(54) **Natural human interleukin-2 and production thereof.**

(57) A natural human interleukin-2 is produced by cultivating human cells capable of producing interleukin-2 in the presence of an inducer, and the interleukin-2 thus produced is useful as cell growth factors or drugs for the prevention or treatment of tumors or treatment of immunodeficiency diseases in warm-blooded animals.

EP 0 132 359 A2

- 1 -

## NATURAL HUMAN INTERLEUKIN-2 AND PRODUCTION THEREOF

This invention relates to natural human interleukin-2 and a method of producing the same.

In the present specification, claims and drawings, interleukin-2 is sometimes abbreviated as IL-2.

Interleukin-2 [formerly called T cell growth factor (TCGF)] is a lymphokine produced by T cells upon stimulation, e.g. with a lectin or alloantigen [Science, 193, 1007 (1976); Immunological Reviews, 51, 257 (1980)]. IL-2 enables long-term in vitro passage of T cells with their functions retained. In addition, IL-2 reportedly promotes thymocyte mitogen reaction (costimulator), restores the antibody production against T cell-dependent antigens by nude mouse spleen cells (T cell-replacing factor) and promotes the differentiation and growth of killer cells (killer helper factor). [The Journal of Immunology, 123, 2928 (1979); Immunological Reviews, 51, 257 (1980)].

By the use of IL-2, a number of killer T cells, helper T cells, and natural killer cells, among others, have been cloned so far [e.g. Nature, 268, 154 (1977); The Journal of Immunology, 130, 981 (1983)]. IL-2 can be used in selective in vitro amplification of antigen-specific killer T cells which can recognize specific antigens, for example, tumor antigens, and destruct tumor cells carrying those specific antigens. It is possible to suppress or inhibit tumor growth by adoptively transferring such tumor-specific killer T cells into tumor-bearing animals [The Journal of Immuno-

0132359

- 2 -

logy, 125, 1904 (1980)].  Furthermore, it is known that IL-2 induces the production of interferon γ [The Journal of Immunology, 130, 1784 (1983)] and activates natural killer cells [The Journal of Immunology, 130, 1970 (1983)].

These experimentally found facts suggest the potential of IL-2 as an antitumor agent.  It is further known that IL-2 restores the helper T cell function in nude mice which are deficient in thymus functions [European Journal of Immunology, vol. 10, page 719 (1980)] and restores the induction of killer T cells against allogenic cells [Nature, vol. 284, page 278 (1980)], and therefore the use of IL-2 in the treatment of immunodeficiency diseases can also be expected to be fruitful.

The present inventors found that cultivation, in the presence of an inducer, of cells capable of producing human IL-2 led to formation and accumulation, in the culture fluid, of at least two IL-2 species differing in properties and succeeded in isolating the two human IL-2 species, namely human IL-2 A and B.  The two human IL-2 species are characterized by the physicochemical properties and biological activities to be described later in detail.  They are human IL-2 species discovered for the first time by the present inventors.

As a result of continued intensive study, the present inventors established a method of producing human IL-2 efficiently by cultivating human IL-2-producing cells in the presence of an inducer and isolating the two human IL-2 species A and B from the culture fluid, and a method of producing human IL-2A-1, A-2, B-1, B-2 and a mixture thereof by subjecting said human IL-2A and B to further purification procedure.

Thus, the present invention provides (1) human interleukin-2 A, B, a mixture thereof, A-1, A-2, B-1, B-2 or a mixture thereof, (2) a method of producing human interleukin-2 A, B, a mixture thereof, A-1, A-2, B-1, B-2 or a mixture thereof, which comprises cultivating, in the presence of an inducer, cells capable of producing human IL-2, thereby causing formation and accumulation of at least one of the human IL-2 and recovering the same.

The cells to be used for the production of human IL-2 may be any cells capable of producing human IL-2. From the viewpoint of availability, however, human lymphocytic or leukocytic cells are preferred. In particular, the use of human peripheral blood-derived lymphocytes is advantageous. It is also possible to use an adequate established cell line. Human peripheral blood-derived lymphocytes can be separated from the buffy coat from normal humans by the specific gravity centrifugation method such as the dextran method or the Ficoll-Hypaque method [Scandinavian Journal of Clinical and Laboratory Investigations, vol. 21, Supplement 97, page 77 (1968)].

As an established leukocyte cell line, there may be used, for instance, a human T leukemia cell line such as JURKAT-FHCRC. The JURKAT-FHCRC cell line is readily available from the Salk Institute Cell Bank (California, USA) or other institutions.

The medium to be used in growing human IL-2-producing cells may be of any type provided that said cells can produce human IL-2 in the medium. Nevertheless, media suited for animal cell culture, for instance commercially available RPMI 1640 medium [Journal of American Medical Association, vol. 199, page 519 (1967)], can be advantageously used. It is preferable to add 0.05 to 1 mg/ml of an antibiotic, such as kanamycin, penicillin or streptomycin. Although it is usual to add 0.1 to 50w/w%, preferably 2 to 20w/w% animal serum, such as fetal bovine serum or bovine serum, it is by far preferable to cultivate the cells in the medium without addition of any animal serum because it makes purification work easier.

As the IL-2 inducer, there may be mentioned, among others, lectins [concanavalin A (Con A), phytohemagglutinin A (PHA)], various antigens including alloantigens, and phorbol esters [12-O-tetradecanoylphorbol-13-acetate (TPA)]. The mixture of these inducers may be added to the medium.

The above-mentioned antigen includes, among others, alloantigens [e.g. allogeneic or xenogeneic B lymphocytic cells with cell division suppressed by treatment with mitomycin C (generally in a concentration of 10 to 200 µg/ml for 30 minutes to 5 hours) or X-ray irradiation [in the case of human cells, for instance, Namalva cells (Intervirology, 5, 205 (1975)), BALL-1 cells (Nature, 267, 843 (1977)), and RPMI 8226 cells (Immunology, 38, 63 (1979))] can be effectively used.

Lectins, phorbol esters and alloantigens are examples of inducers for combined use.. More concretely, it is preferable to use, for instance, Con A as lectin, TPA as phorbol ester and Namalva cells as alloantigen in concentrations of, for example, 5 to 80·µg/ml, 1 to 1,500 ng/ml and $1 \times 10^4$ to $5 \times 10^6$·cells/ml, respectively.

The cultivation is performed in the manner of static culture, spinner culture, roller bottle culture and so on. Spinner culture is preferable for IL-2 production by large-scale cultivation in serum-containing medium, whereas static culture, especially in a multistage tray, is suited for efficient production of IL-2 in a serum-free medium. Inoculation is conducted generally in a cell concentration of 0.1 to $50 \times 10^6$ cells/ml, preferably 1 to 5 $\times 10^6$ cells/ml, and incubation is conducted at 30 to 40°C in the presence of 1 to 20% $CO_2$.

pH of the culture medium during the cultivation is, generally maintained between 6 and 8, preferably between 7 and 7.4.

The incubation period is selected on the basis of induction and production of IL-2. Generally, it is preferable to separate and collect the culture supernatant after 10 to 120 hours, more preferably 48 to 96 hours, of incubation.

An IL-2-dependent cell line may be used for assaying human IL-2 accumulated in the medium according to the present invention. Since IL-2 is known to promote the growth of IL-2-dependent cells not only of humans but also of rats and mice, among others [Immunological Reviews, 51, 257 (1980)], not only IL-2-dependent human cell lines but also IL-2-dependent rat or mouse cell lines can also be used [Journal of Immunology, 130, 981 and 988 (1983)].

Separation of the IL-2-species from the culture supernatant and purification thereof according to the present invention can be carried out by adequate combined use of known purification methods, which include, among others, methods making use of solubility differences, such as salting out and precipitation from a solution, methods making use of differences in molecular weight, such as ultrafiltration, gel filtration and SDS-polyacrylamide gel electrophoresis, methods making use of differences in electric charge such as ion exchange chromatography, methods making use of specific affinity, such as affinity chromatography, methods making use of differences in hydrophobicity such as reverse phase high performance liquid chromatography, and methods making use of differences in isoelectric point, such as isoelectric focusing.

Thus, for instance, an IL-2 containing culture supernatant is collected by centrifugation and made acidic (pH 3.5) with a mineral acid such as concentrated hydrochloric acid, the resulting precipitate is removed by using filter paper, for instance, the filtrate is passed over a cation exchange chromatographic carrier such as sulfopropyl-crosslinked dextran, e.g. SP-Sephadex® (Pharmacia, Sweden) column, and elution is carried out with a salt (e.g. NaCl)-containing buffer. The active fraction thus collected is dialyzed and then passed through an anion exchange chromatographic carrier, such as a DEAE-Sephacel® (Pharmacia, Sweden) column, followed by elution with a salt (e.g. NaCℓ)-containing buffer.

Then the active eluate is concentrated by using an ultrafiltration apparatus and the active eluate is subjected to gel filtration using polyacrylamide-agarose, e.g. Ultrogel® (LKB, Sweden) column or the like.

The active fraction collected is dialyzed and again treated with an anion exchanger such as DEAE-cellulose, elution being carried out with a salt solution (for example elution making use of a linear NaCl concentration gradient). In this way, a mixture of human IL-2A and B is obtained. The ratio of IL-2A to IL-2B ranges between 1:1 and 1:3 (by weight).

The active fraction is again subjected to gel filtration using polyacrylamide-agarose e.g. Ultrogel® column.

In the above separation and purification process, the elution of IL-2 with a salt solution (e.g. a buffer containing 0 to 0.2 M NaCl) following adsorption on an anion exchanger such as DEAE-cellulose, when conducted with a gradual salt concentration gradient, gives two activity peaks of IL-2. The first activity peak fraction collected during elution at the lower salt concentration range is called a human IL-2A-containing fraction, while the second activity peak fraction collected during elution at the higher salt concentration range is called a human IL-2B-containing fraction. In this way, there can be obtained crude human IL-2A or B.

When subjected to high performance liquid chromatography, thus-obtained crude IL-2A fraction can be resolved into two peaks of activity, IL-2A-1 and IL-2A-2, each homogeneous on SDS-polyacrylamide gel electrophoresis. Two peaks of activity can be collected together to give IL-2A or collected separately to give IL-2A-1 and IL-2A-2, repectively. Human IL-2A is thus a mixture of IL-2A-1 and IL-2A-2. The ratio of IL-2A-1 to IL-2A-2 ranges between 1:1 and 1:2 (by weight).

Similarly, high performance liquid chromatography can resolve the crude IL-2B fraction into two peaks of activity, IL-2B-1 and IL-2B-2, each homogeneous on SDS-polyacrylamide gel electrophoresis. Two peaks of activity can be collected together to give IL-2B or collected separately to give IL-2B-1 and IL-2B-2, respectively. IL-2B is thus a mixture of IL-2B-1 and IL-2B-2. The ratio of IL-2B-1 to IL-2B-2 ranges between 1:1 and 1:2 (by weight).

The above procedures are carried out at a temperature ranging from 0 to 30°C, preferably 2 to 6°C.

As the high performance liquid chromatography, reverse-phase high performance liquid chromatography is advantageously employed. The temperature is 20 to 40°C, preferably 25 to 35°C.

By DEAE-Sephacel$^R$ column chromatography in the purification procedure described above, IL-2 is eluted as a single peak of activity containing IL-2A and IL-2B. When the thus obtained fraction containing both IL-2A and IL-2B is subjected to reverse-phase high performance liquid chromatography, IL-2 can be resolved into two peaks of activity, one containing IL-2A-1 and IL-2B-1, and the other IL-2A-2 and IL-2B-2. Therefore, two peaks of activity can be collected together to give a mixture of IL-2A and IL-2B or collected separately to give a mixture of IL-2A-1 and IL-2B-1, and a mixture of IL-2A-2 and IL-2B-2, respectively. The ratio of IL-2A-1 to IL-2B-1 ranges between 1:1 and 1:3 (by weight), and the ratio of Il-2A-2 to IL-2B-2 ranges between 1:1 and 1:3 (by weight).

Thus-obtained human IL-2 sample can be assayed for purity by various electrophoretic methods, among others. Particularly preferred is SDS-polyacrylamide gel electrophoresis, in which the purity of protein can be determined with good sensitivity by the Coomassie brilliant blue (Sigma Chemical Company, USA) or silver staining method, for instance. In SDS-polyacrylamide gel electrophoresis, it is also possible to calculate the molecular weight of the active entity.

The human IL-2 of the present invention is separated and recovered in accordance with the present invention from a culture of cells capable of producing human IL-2. In addition, it is also obtained in large amounts by utilizing the gene manipulation technique. Furthermore, monoclonal or polyclonal antibodies to said human IL-2 can be produced by immunizing mice, rabbits or other animals with the human IL-2 of the present invention as an immunogen, and the antibodies obtained in this manner can be used in preparing affinity columns for the isolation and recovery of the human IL-2 of the present invention.

The human IL-2 obtainable in accordance with the present invention is low in toxicity, has little antigenecity because of its origin in humans, exhibits activity of maintaining the functions of normal T cells and natural killer cells, and therefore, can be used in *in vitro* long-term culture, subculture or cloning of T cells and natural killer cells.

Furthermore, the IL-2 of the present invention can selectively promote *in vitro* proliferation of, for instance, antigen-specific killer T cells, which recognize and destruct cells with tumor antigens, or natural killer cells, which are capable of killing tumors without prior sensitization with the tumor antigens. Furthermore, when the human IL-2 of the present invention is used for inoculation of a living organism simultaneously with the transplantation of said killer T cells, the antitumor effect of the killer cells is increased. Therefore, the present human IL-2 is useful as growth-promoting agents for killer T cells and natural killer cells, as antitumor agents, or as immunodeficiency disease-treating agents. The present human IL-2 is useful for the prevention or treatment of tumor or treatment of immunodeficiency diseases in warm-blooded animals (e.g. mouse, rat, rabbit, dog, cat, pig, horse, sheep, cattle, human). For this purpose, the human IL-2 of the present invention can be mixed or diluted with a *per se* known and pharmaceutically acceptable carrier, diluent or excipient to make up a composition in the form of, for instance, injections or capsules. Such a composition can be administered either parenterally or orally. The human IL-2 can be used either alone or in combination with killer T cells or natural killer cells grown *in vitro* in the manner mentioned above.

Very small doses of the human IL-2 according to the present invention are sufficient for the above purposes, since the dissociation constant between the IL-2 and the IL-2 receptor of cells is very small.

For promoting the in vitro growth of T cells, the human IL-2 according to the present invention can be added to the medium in a concentration of 0.01 to 1 U/ml, preferably 0.1 to 0.5 U/ml.

Usually IL-2 (about 0.1 to 0.5 U/ml) is added to a cell suspension containing alloantigen-sensitized T cells which have been obtained by 3-day lymphocyte-mixed culture of human peripheral blood T cells ($1 \times 10^6$ cells/ml) in the presence of X ray-irradiated (1500 rads) B cell transformants ($1 \times 10^6$ cells/ml) in RPMI 1640 medium containing 20% fetal bovine serum at 37°C under an atmosphere of 5% $CO_2$. The incubation is continued for about one month with exchange of medium at approximately weekly intervals.

In the present application, amino acids, when convenient, are abbreviated according to the IUPAC-IUB Commission on Biochemical Nomenclature or the practice in the field of the art. In case optical isomerism is involved, the amino acids are in the L-form unless otherwise specifically indicated.

| Asp | aspartic acid |
|-----|---------------|
| Asn | asparagine |
| Thr | threonine |
| Ser | serine |
| Glu | glutamic acid |
| Gln | glutamine |
| Gly | glycine |
| Ala | alanine |
| Val | valine |
| Met | methionine |
| Ile | isoleucine |
| Leu | leucine |
| Tyr | tyrosine |
| Phe | phenylalanine |
| Lys | lysine |
| His | histidine |
| Arg | arginine |

0132359

- 10 -

| Pro | proline |
| Cys | cystine |
| Trp | tryptophan |
| Asp/Asn | Asp and Asn |
| Glu/Gln | Glu and Gln |

BRIEF DESCRIPTION OF THE DRAWINGS

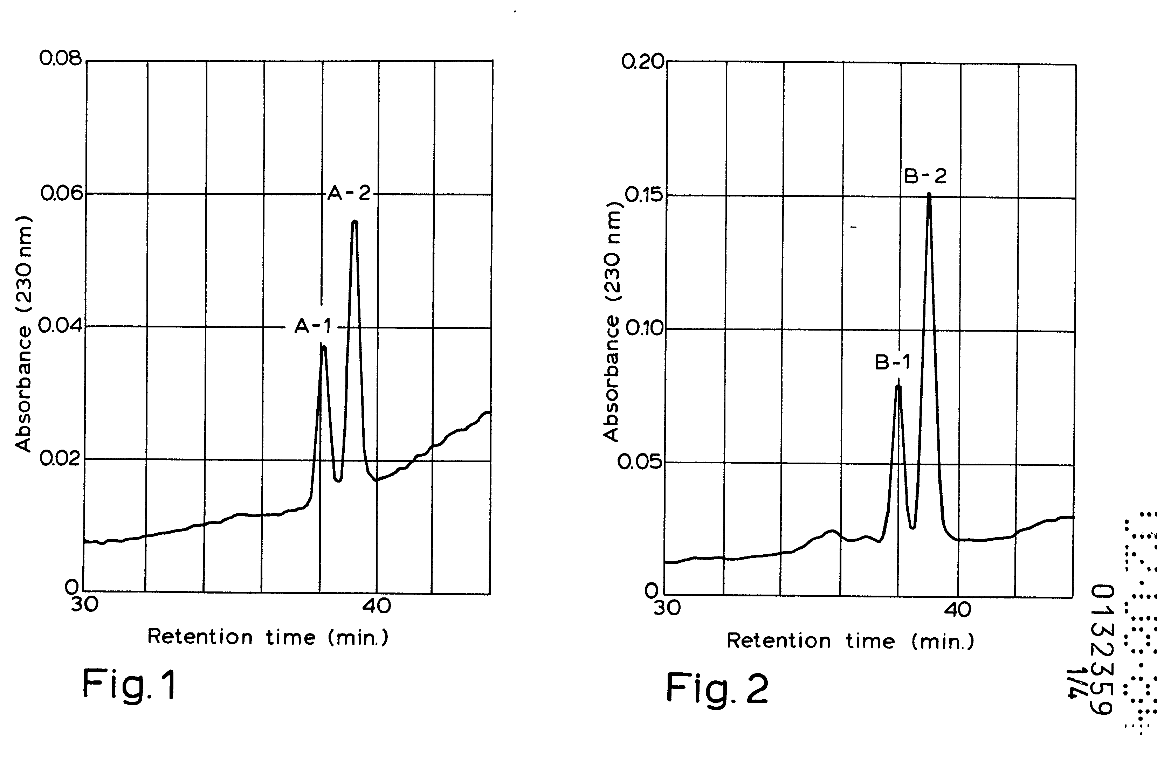

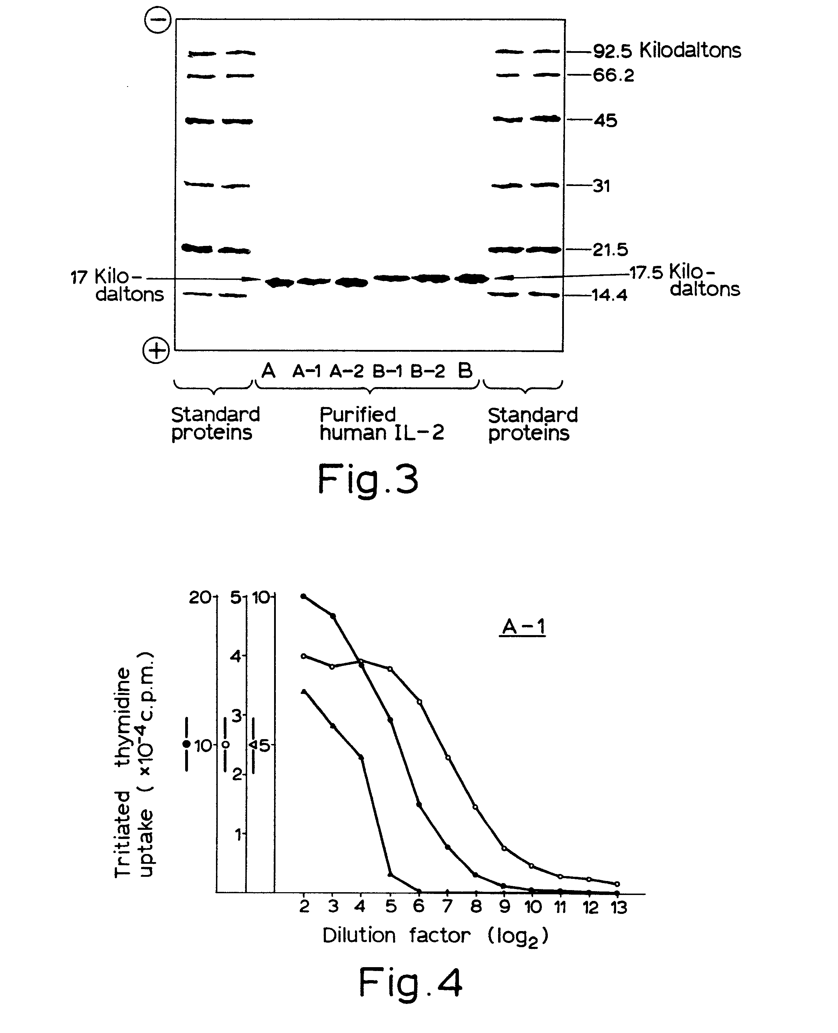

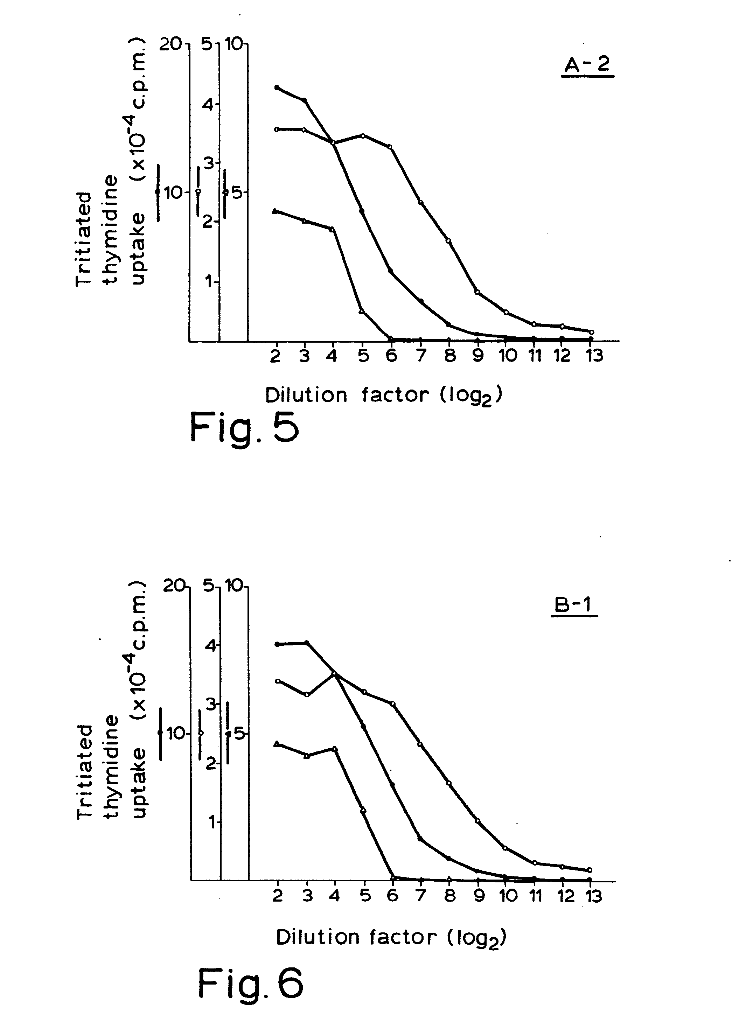

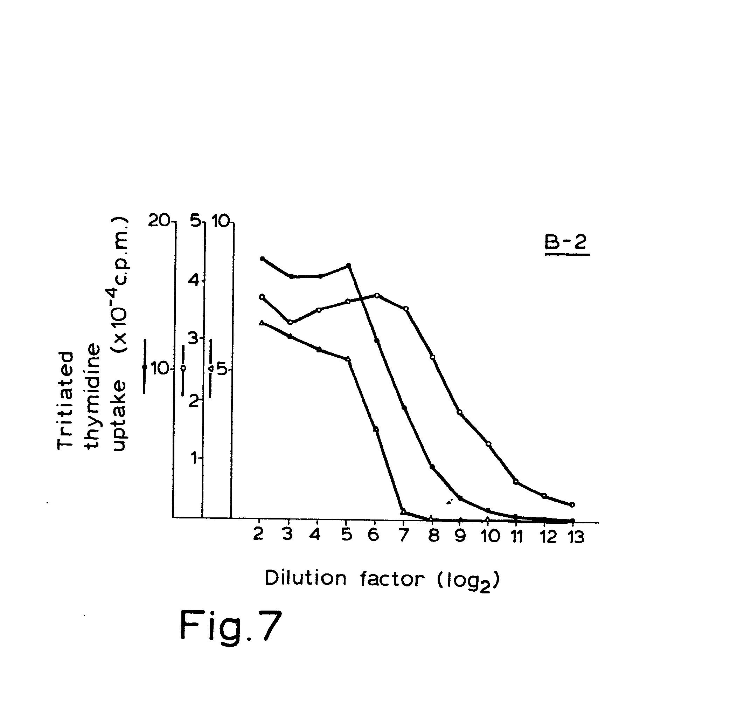

Fig. 1 shows the results of high performance liquid chromatography of human IL-2A-1 and IL-2A-2. Fig. 2 shows the results of high performance liquid chromatography of human IL-2B-1 and IL-2B-2. Fig. 3 shows the results of SDS-polyacrylamide gel electrophoresis of human IL-2. Figs. 4, 5, 6 and 7 illustrate the biological activity of each human IL-2 species upon IL-2-dependent cells.

## Example 1

Production of IL-2 from human peripheral blood lymphocytes:

An equal volume of RPMI 1640 medium is added to the peripheral blood buffy coat collected from 30 to 50 normal subjects. After incubating the mixture at 37°C in a roller bottle overnight, dextran (Meito Sangyo Co. Ltd., Japan, molecular weight 300,000-500,000) is added at a concentration of 1%, and the whole mixture is stood still for 30 min to isolate peripheral blood lymphocytes. Thus obtained peripheral blood lymphocytes are suspended in RPMI 1640 medium at a concentration of $5 \times 10^6$ cells/ml. To the suspension are added 15 ng/ml of TPA, 20 µg/ml of Con A, and Namalva cells which have been treated with 80 µg/ml of mitomycin C (Kyowa Hakko Kogyo Co., Ltd. Japan) for 30 minutes, followed by incubation at 37°C under 5% $CO_2$ in air for 72 hours in a Cell Factory (Nunc, Denmark).

## Example 2

Isolation and properties of IL-2A, IL-2A-1 and IL-2A-2:

[1] The culture supernatant obtained by following the procedure described in Example 1 is adjusted to pH 3.5 by the addition of hydrochloric acid. The resulting precipitate is filtered off using Toyo No. 2 filter paper (Toyo Chemical Co. Ltd., Japan). The filtrate is applied to an SP-Sephadex®

C-25 (Pharmacia, Sweden) column (500 ml in volume) equilibrated with 0.02 M citrate buffer (pH 3.5) for adsorption of IL-2. Elution is performed with 0.02 M citrate buffer (pH 3.5) containing 2 M NaCl. Active fractions are combined, dialyzed against 0.01 M Tris-HCl buffer (pH 8.0), and the dialysate is applied to a DEAE-Sephacel® (Pharmacia, Sweden) column (200 ml in volume) equilibrated with 0.01 M Tris-HCl buffer (pH 8.0) for IL-2 adsorption. Elution is performed by applying a linear gradient of NaCl concentration (0 to 0.2 M NaCl). Active fractions are pooled and concentrated about 50-fold using a Diaflo® YM-5 membrane (Amicon Corporation, USA). The concentrated solution containing IL-2 is subjected to gel filtration using an Ultrogel® AcA 54 (LKB, Sweden) column. (442 ml in volume) equilibrated with 0.1 M Tris-HCl buffer (pH 8.0) containing 1 M NaCl. Active fractions are pooled and dialyzed against 0.01 M Tris-HCl buffer (pH 8.0) and the dialysate is applied to a DE-52 (DEAE-cellulose; Whatman, Great Britain) column (20 ml in volume) equilibrated with 0.01 M Tris-HCl buffer (pH 8.0) for IL-2 adsorption. Elution of IL-2 is performed by applying a linear gradient of NaCl concentration (0 to 0.2 M NaCl). By this step IL-2 is resolved into two peaks of activity. The first activity peak fractions eluted at a lower NaCl concentration range (0.040 to 0.055 M NaCl) are combined to give a human IL-2A-containing solution.

The IL-2A-containing solution is concentrated with a Diaflo® YM-5 membrane (Amicon Corporation, U.S.A.) and subjected to the second gel filtration using an Ultrogel® AcA 54 column. The active fractions are pooled and subjected to reverse phase high performance liquid chromatography using an Ultrapore® RPSC (Altex, U.S.A.) column. Elution is performed using trifluoroacetic acid-acetonitrile as a mobile phase to give two peaks of activity. Two peaks are collected together to give IL-2A. The recovery of IL-2 activity is 2.8%. Following the same procedure the first and the second peak of activity are pooled separately to give IL-2A-1 and IL-2A-2, respectively. The recovery of IL-2 activity from the starting material is 1.1% for IL-2A-1 and 1.5% for IL-2A-2.

The conditions used for high performance liquid chromatography are as follows. Column, Ultrapore®RPSC (4.6 x 75 mm); column temperature, 30°C; solvent A, 0.1% trifluoroacetic acid-99.9% water; solvent B, 0.1% trifluoroacetic acid-99.9% acetonitrile; elution program, O min (68% A + 32% B) - 25 min (55% A + 45% B) - 35 min (45% A + 55% B) - 45 min (30% A + 70% B) - 48 min (100% B); flow rate, 0.8 ml/min; monitor, 230 nm. Under the above conditions the retention time is 38 ± 0.4 min for IL-2A-1 and 39 ± 0.4 min for IL-2A-2. The elution pattern of high performance liquid chromatography is shown in Fig. 1.

[2] Properties of IL-2A

The IL-2A preparation obtained in [1] is examined for the following characteristics:

(1) Homogeneity

The IL-2 preparation migrates as a single band on SDS-polyacrylamide gel electrophoresis under reducing and non-reducing conditions. Gel electrophoresis is performed according to the method of Laemmli et al. [Nature, vol. 227, page 680 (1970)] followed by silver staining (Bio-Rad) (Fig. 3). The mobility of IL-2A under reducing conditions is the same as that under nonreducing conditions.

(2) Molecular weight

The molecular weight of said IL-2A preparation is calculated to be 17 ± 1 kilodaltons based on the results of SDS-polyacrylamide gel electrophoresis (Fig. 3).

(3) Isoelectric point

Isoelectric focusing is performed using Pharmalite® pH 5-8 (Pharmacia, Sweden) in Sephadex® IEF (Pharmacia, Sweden) gel in the presence of inactivated fetal bovine serum and polyethylene glycol. Said IL-2A preparation is electrophoresed as a single band corresponding to the isoelectric point of 7.2 ± 0.2.

(4)  Amino acid composition

An aliquot (0.5 to 1.0 µg) of said IL-2A preparation is dissolved in 0.1 ml of 6 N hydrochloric acid containing 4% (v/v) thioglycolic acid and subjected to hydrolysis in a sealed tube at 110°C for 24, 48 or 72 hours.  Thereafter, hydrochloric acid is removed under reduced pressure, 0.30 to 0.35 ml of 0.02 N hydrochloric acid is added, and the resulting mixture is used as a sample for amino acid analysis. Amino acid analysis is performed in a Hitachi model 835 amino acid analyzer (Hitachi Ltd., Japan) by the fluorescence method using orthophthalaldehyde.  The results are shown in Table 1.

Table 1

| Amino acid | Content (mole %) |
|---|---|
| Asp/Asn | 9.1 ± 1.9 |
| Thr | 8.8 ± 2.6 |
| Ser | 7.3 ± 1.5 |
| Glu/Gln | 14.3 ± 3.0 |
| Gly | 5.5 ± 5.4 |
| Ala | 4.9 ± 1.0 |
| Val | 3.6 ± 0.7 |
| Met | 3.2 ± 0.7 |
| Ile | 6.1 ± 1.8 |
| Leu | 14.9 ± 4.4 |
| Tyr | 2.3 ± 0.5 |
| Phe | 6.8 ± 4.1 |
| Lys | 7.9 ± 1.7 |
| His | 2.1 ± 0.4 |
| Arg | 3.3 ± 0.8 |

The content values given in Table 1 are expressed in terms of mole percent based on the total number of amino acid residues except Pro, 1/2 Cys, and Trp.

(5)  Amino terminal amino acid sequence

A 10µg  portion (0.6 nmol) of said IL-2A preparation

- 14 -

is analyzed for the amino terminal amino acid sequence by automated Edman degradation using a gas phase protein sequenator (model 470A, Applied Biosystems, USA). Phenyl-thiohydantoin-amino acids are identified and quantitated by high performance liquid chromatography using a Micro-pack SP-ODS column (Varian, USA). The amino terminal amino acid sequence (residues from No. 1 through No. 13) is shown below.

Formula

```
    1   2   3   4   5   6   7 8  9 10 11  12   13
H-Ala-Pro-X-Ser-Ser-Ser-X-X-Lys-X-Gln-Leu-Gln
```

wherein each X is yet unknown.

(6) Elution pattern on high performance liquid chromatography

When subjected to reverse phase high performance liquid chromatography under the same conditions as those described in [1], said IL-2A preparation is resolved into two peaks corresponding to IL-2A-1 and IL-2A-2, respectively.

[3] Properties of purified IL-2A-1

The IL-2A-1 preparation obtained in [1] is examined for the following properties:

(1) Homogeneity

When subjected to the same procedure as that described in Example 2 [2] (1), said IL-2A-1 preparation migrates as a single band on SDS-polyacrylamide gel electrophoresis (Fig. 3). The mobility of IL-2A-1 under reducing conditions is the same as that under non-reducing conditions.

(2) Molecular weight

The molecular weight of said IL-2A-1 preparation is calculated to be $17 \pm 1$ kilodaltons based on the results of SDS-polyacrylamide gel electrophoresis (Fig. 3).

(3) Isoelectric point

When subjected to the same procedure as that described in Example 2 [2] (3), said IL-2A-1 preparation is electrophoresed as a single band corresponding to the isoelectric point of $7.2 \pm 0.2$.

(4)   Amino acid composition

The amino acid composition of IL-2A-1 is determined by the same method as that described in Example 2 [2] (4). The results are shown in Table 2.

Table 2

| Amino acid | Content (mole %) |
|---|---|
| Asp/Asn | 9.2 ± 1.9 |
| Thr | 8.5 ± 2.6 |
| Ser | 7.3 ± 1.5 |
| Glu/Gln | 14.6 ± 3.0 |
| Gly | 6.7 ± 5.4 |
| Ala | 4.9 ± 1.0 |
| Val | 3.5 ± 0.7 |
| Met | 3.5 ± 0.7 |
| Ile | 6.1 ± 1.3 |
| Leu | 15.0 ± 3.0 |
| Tyr | 2.4 ± 0.5 |
| Phe | 4.8 ± 1.0 |
| Lys | 7.8 ± 1.6 |
| His | 2.2 ± 0.5 |
| Arg | 3.6 ± 0.8 |

The content values given in Table 2 are expressed in terms of mole percent based on the total number of amino acid residues except Pro, 1/2 Cys, and Trp.

(5)   Amino terminal amino acid sequence

The amino terminal amino acid sequence of IL-2A-1 is determined by the same method  as that used in Example 2 [2] (5).  The amino terminal amino acid sequence (residues from No. 1 through No. 12) is shown below:

Formula

```
    1    2    3    4    5    6   7 8 9 10 11   12
H-Ala-Pro-X-Ser-Ser-Ser-X-X-X-X-Gln-Leu
```

wherein each X is yet unknown.

[4]   Properties of IL-2A-2

The IL-2A-2 preparation obtained in [1] is examined for the following characteristics:

(1) Homogeneity

When tested by the same procedure as that described in [2] (1), said IL-2A-2 preparation gives a single band on SDS-polyacrylamide gel electrophoresis (Fig. 3). The mobility of IL-2A-2 under reducing conditions is the same as that under non-reducing conditions.

(2) Molecular weight

The molecular weight of said IL-2A-2 preparation is calculated to be 17 ± 1 kilodaltons based on the results of SDS-polyacrylamide gel electrophoresis (Fig. 3).

(3) Isoelectric point

When subjected to the same procedure as that described in [2] (3), the IL-2A-2 preparation is electrophoresed as a single band corresponding to the isoelectric point of 7.2 ± 0.2.

(4) Amino acid composition

The amino acid composition of IL-2A-2 is determined by the same method as that described in [2] (4). The results are shown in Table 3.

Table 3

| Amino acid | Content (mole %) |
| --- | --- |
| Asp/Asn | 9.1 ± 1.9 |
| Thr | 8.9 ± 1.8 |
| Ser | 7.2 ± 1.5 |
| Glu/Gln | 14.2 ± 2.9 |
| Gly | 4.9 ± 4.1 |
| Ala | 4.5 ± 0.9 |
| Val | 3.6 ± 0.7 |
| Met | 3.0 ± 0.6 |
| Ile | 6.1 ± 1.8 |
| Leu | 14.8 ± 4.4 |
| Tyr | 2.3 ± 0.5 |
| Phe | 7.8 ± 4.1 |
| Lys | 8.0 ± 1.7 |

| Amino acid | Content (mole %) |
|------------|-------------------|
| His | 2.1 ± 0.4 |
| Arg | 3.2 ± 0.7 |

The content values given in Table 3 are expressed in terms of mole percent based on the total number of amino acid residues except Pro, 1/2 Cys, and Trp.

(5) Amino terminal amino acid sequence

The amino terminal amino acid sequence of IL-2A-2 is determined by the same method as that described in [2] (5). The amino terminal amino acid sequence (residues from No. 1 through No. 13) is shown below.

Formula

```
  1   2   3   4   5   6  7 8  9 10 11  12  13
H-Ala-Pro-X-Ser-Ser-Ser-X-X-Lys-X-Gln-Leu-Gln
```

wherein each X is yet unknown.

## Example 3

Isolation and properties of IL-2B, IL-2B-1 and IL-2B-2:

[1] Following the procedure of Example 2, an IL-2-containing human peripheral blood lymphocyte culture supernatant is treated. IL-2 elution from the DE-52 column is carried out by applying a linear NaCl concentration gradient (0 to 0.2 M NaCl), and the second activity peak fractions eluted at a higher NaCl concentration range (0.055 to 0.075 M NaCl) are combined to give an IL-2B-containing solution.

The IL-2B-containing solution is concentrated with a Diaflo® YM-5 membrane (Amicon Corporation, U.S.A.) and subjected to the second gel filtration using an Ultrogel AcA 54 column. The active fractions are pooled and subjected to reverse phase high performance liquid chromatography using an Ultrapore® RPSC (Altex, U.S.A.) column. Elution is performed using trifluoroacetic acid-acetonitrile as a mobile phase to give two peaks of activity. Two peaks are collected together to give IL-2B. The recovery of IL-2 activity is 6.3%. Following the same procedure the first and the second peak of activity are pooled separately to give IL-2B-1 and IL-2B-2, respectively. The recovery of IL-2 activity from the starting material is 2.3% for IL-2B-1 and 3.6% for IL-2B-2.

The conditions used for high performance liquid chromatography are as follows. Column, Ultrapore[R] RPSC (4.6 x 75 mm); column temperature, 30°C; solvent A, 0.1% trifluoroacetic acid-99.9% water; solvent B, 0.1% trifluoroacetic acid-99.9% acetonitrile; elution program, 0 min (68% A + 32% B)-25 min (55% A + 45% B)-35 min (45% A + 55% B)-45 min (30% A + 70% B)-48 min (100% B); flow rate, 0.8 ml/min; monitor, 230 nm. Under the above conditions the retention time is 38 ± 0.4 min for IL-2B-1 and 39 ± 0.4 min for IL-2B-2. The elution pattern of high performance liquid chromatography is shown in Fig. 2.

[2] Properties of purified IL-2B

The IL-2B preparation obtained in [1] is examined for the following properties:

(1) Homogeneity

When subjected to the same procedure as that described in Example 2 [2] (1), said IL-2B preparation migrates as a single band on SDS-polyacrylamide gel electrophoresis (Fig. 3). The mobility of IL-2 B under reducing conditions is the same as that under non-reducing conditions.

(2) Molecular weight

The molecular weight of said IL-2B preparation is calculated to be 17.5 ± 1 kilodaltons based on the results of SDS-polyacrylamide gel electrophoresis (Fig. 3).

(3) Isoelectric point

When subjected to the same procedure as that described in Example 2 [2] (3), said IL-2B preparation is electrophoresed as a single band corresponding to the isoelectric point of 6.6 ± 0.2.

(4) Amino acid composition

The amino acid compositon of IL-2B is determined by the same method as that described in Example 2 [2] (4). The results are shown in Table 4.

## Table 4

| Amino acid | Content (mole %) |
|---|---|
| Asp/Asn | 9.6 ± 2.0 |
| Thr | 9.5 ± 2.0 |
| Ser | 6.4 ± 1.3 |
| Glu/Gln | 14.7 ± 3.0 |
| Gly | 4.1 ± 4.0 |
| Ala | 4.7 ± 1.5 |
| Val | 3.8 ± 1.4 |
| Met | 3.1 ± 0.7 |
| Ile | 6.3 ± 1.8 |
| Leu | 15.4 ± 4.4 |
| Tyr | 2.5 ± 0.5 |
| Phe | 6.0 ± 1.8 |
| Lys | 8.7 ± 1.8 |
| His | 1.9 ± 0.7 |
| Arg | 3.2 ± 0.7 |

The content values given in Table 4 are expressed in terms of mole percent based on the total number of amino acid residues except Pro, 1/2 Cys, and Trp.

(5)   Amino terminal amino acid sequence

The amino terminal amino acid sequence of IL-2B is determined by the same method  as that used in Example 2 [2] (5).   The amino terminal amino acid sequence (residues from No. 1 through No. 20) is shown below:

Formula

1   2   3   4   5   6   7   8   9  10  11  12  13  14  15 16
H-Ala-Pro-X-Ser-Ser-Ser-X-Lys-Lys-X-Gln-Leu-Gln-Leu-Glu-X-
17  18  19  20
Leu-Leu-Leu-Asp

wherein each X is yet unknown.

(6)   Elution pattern on high performance liquid chromatography

When subjected to reverse phase high performance liquid chromatography under the same conditions as those described in [1], said IL-2B preparation is resolved into two peaks corresponding to IL-2B-1 and IL-2B-2, respectively.

[3] Properties of purified IL-2B-1

The IL-2B-1 preparation obtained in [1] is examined for the following properties:

(1) Homogeneity

When subjected to the same procedure as that described in Example 2 [2] (1), said IL-2B-1 preparation migrates as a single band on SDS-polyacrylamide gel electrophoresis (Fig. 3). The mobility of IL-2B-1 under reducing conditions is the same as that under nonreducing conditions.

(2) Molecular weight

The molecular weight of said IL-2B-1 preparation is calculated to be 17.5 ± 1 kilodaltons based on the results of SDS-polyacrylamide gel electrophoresis (Fig. 3).

(3) Isoelectric point

When subjected to the same procedure as that described in Example 2 [2] (3), said IL-2B-1 preparation is electrophoresed as a single band corresponding to the isoelectric point of 6.6 ± 0.2.

(4) Amino acid composition

The amino acid composition of IL-2B-1 is determined by the same method as that described in Example 2 [2] (4). The results are shown in Table 5.

### Table 5

| Amino acid | Content (mole %) |
| --- | --- |
| Asp/Asn | 9.5 ± 1.9 |
| Thr | 9.1 ± 1.9 |
| Ser | 6.4 ± 1.3 |
| Glu/Gln | 14.6 ± 3.0 |
| Gly | 4.9 ± 4.0 |
| Ala | 5.0 ± 1.5 |
| Val | 4.5 ± 1.4 |
| Met | 2.9 ± 0.6 |

| Amino acid | Content (mole %) |
|------------|------------------|
| Ile | 6.0 ± 1.8 |
| Leu | 14.6 ± 4.4 |
| Tyr | 2.5 ± 0.5 |
| Phe | 6.1 ± 1.8 |
| Lys | 8.7 ± 1.8 |
| His | 1.8 ± 0.7 |
| Arg | 3.2 ± 0.7 |

The content values given in Table 5 are expressed in terms of mole percent based on the total number of amino acid residues except Pro, 1/2 Cys, and Trp.

(5) Amino terminal amino acid sequence

The amino terminal amino acid sequence of IL-2B-1 is determined by the same method as that used in Example 2 [2] (5). The amino terminal amino acid sequence (residues from No. 1 through No. 14) is shown below:

Formula

```
   1   2   3   4   5   6   7   8   9  10  11  12  13  14
H-Ala-Pro-X-Ser-Ser-Ser-X-Lys-Lys-X-Gln-Leu-Gln-Leu
```

wherein each X is yet unknown.

[4] Properties of purified IL-2B-2

The IL-2B-2 preparation obtained in [1] is examined for the following properties.

(1) Homogeneity

When subjected to the same procedure as that described in Example 2[2](1) above, said IL-2B-2 preparation migrates as a single band on SDS-polyacrylamide gel electrophoresis (Fig. 3). The mobility of IL-2B-2 under reducing conditions is the same as that under nonreducing conditions.

(2) Molecular weight

The molecular weight of said IL-2B-2 preparation is calculated to be 17.5 ± 1 kilodaltons based on the results of SDS-polyacrylamide gel electrophoresis (Fig. 3).

(3) Isoelectric point

When subjected to the same procedure as that described

0132359

- 22 -

in Example 2[2](3), said IL-2B-2 preparation is electrophoresed as a single band corresponding to the isoelectric point of 6.6 ± 0.2.

(4) Amino acid composition

The amino acid composition of IL-2B-2 is determined by the same method as that described in Example 2 [2](4). The results obtained are shown in Table 6.

Table 6

| Amino acid | Content (mole %) |
|---|---|
| Asp/Asn | 9.7 ± 2.0 |
| Thr | 9.7 ± 2.0 |
| Ser | 6.4 ± 1.3 |
| Glu/Gln | 14.7 ± 3.0 |
| Gly | 3.7 ± 3.0 |
| Ala | 4.5 ± 0.9 |
| Val | 3.5 ± 0.7 |
| Met | 3.2 ± 0.7 |
| Ile | 6.4 ± 1.3 |
| Leu | 15.8 ± 3.2 |
| Tyr | 2.5 ± 0.5 |
| Phe | 6.0 ± 1.8 |
| Lys | 8.7 ± 1.8 |
| His | 2.0 ± 0.6 |
| Arg | 3.2 ± 0.7 |

The content values given in Table 6 are expressed in terms of mole percent based on the total number of amino acid residues except Pro, 1/2 Cys, and Trp.

(5) Amino terminal amino acid sequence

The amino terminal amino acid sequence of IL-2B-2 is determined by the same method as that used in Example 2 [2] (5). The amino terminal amino acid sequence (residues from No. 1 through No. 20) is shown below:

Formula

```
   1   2   3   4   5   6   7   8   9  10 11  12  13  14  15
H-Ala-Pro-X-Ser-Ser-Ser-X-Lys-Lys-X-Gln-Leu-Gln-Leu-Glu-
16 17  18  19  20
X-Leu-Leu-Leu-Asp
```

wherein each X is yet unknown.

## Example 4

Isolation and properties of a mixture of IL-2A and IL-2B, a mixture of IL-2A-1 and IL-2B-1, and a mixture of IL-2A-2 and IL-2B-2:

[1]  The culture supernatant obtained by following the procedure described in Example 1 is adjusted to pH 3.5 by the addition of hydrochloric acid.  The resulting precipitate is filtered off using Toyo No. 2 filter paper (Toyo Chemical Co., Ltd., Japan).  The filtrate is applied to an SP-Sephadex® C-25 (Pharmacia, Sweden) column (500 ml in volume) equilibrated with 0.02 M citrate buffer (pH 3.5) for adsorption of IL-2.  Elution is performed with 0.02 M citrate buffer (pH 3.5) containing 2 M NaCl.  Active fractions are combined, dialyzed against 0.01 M Tris-HCl buffer (pH 8.0), and the dialysate is applied to a DEAE-Sephacel® (Pharmacia, Sweden) column (200 ml in volume) equilibrated with 0.01 M Tris-HCl buffer (pH 8.0) for IL-2 adsorption.  Elution is performed by applying a linear gradient of NaCl concentration (0 to 0.2 M NaCl) to give a single peak of activity which contains IL-2A and IL-2B.

The IL-2A- and IL-2B-containing solution thus obtained is concentrated with a Diaflo® YN-5 membrane (Amicon Corporation, U.S.A.) and subjected to gel filtration using an Ultrogel® AcA 54 column. The active fractions are pooled and subjected to reverse phase high performance liquid chromatography using an Ultrapore® RPSC (Altex, U.S.A.) column.  Elution is performed using trifluoroacetic acid-acetonitrile as a mobile phase to give two peaks of activity.  Two peaks are collected together to give a mixture of IL-2A and IL-2B.  The recovery of IL-2 activity is 10.5%.  Following the same procedure the first and the second peak of activity are pooled separately to give a mixture of IL-2A-1 and IL-2B-1, and a mixture of IL-2A-2 and IL-2B-2, respectively. The recovery of IL-2 activity from the starting material is 3.6% for a mixture of IL-2A-1 and IL-2B-1 and 6.4% for a mixture of IL-2A-2 and IL-2B-2.

The conditions used for high performance liquid chromatography are as follows. Column, Ultrapore®RPSC (4.6 x 75 mm); column temperature, 30°C; solvent A, 0.1% trifluoroacetic acid-99.9% water; solvent B, 0.1% trifluoroacetic acid-99.9% acetonitrile; elution program, O min (68% A + 32% B)- 25 min (55% A + 45% B)- 35 min (45% A + 55% B)- 45 min (30% A + 70% B)- 48 min (100% B); flow rate, 0.8 ml/min; monitor, 230 nm. Under the above conditions the retention time is 38 ± 0.4 min for the first peak and 39 ± 0.4 min for the second peak.

[2] Properties of a mixture of IL-2A and IL-2B

The mixture of IL-2A and IL-2B obtained in [1] is examined for the following properties:

(1) Molecular weight

When subjected to the same procedure as that described in Example 2 [2] (1), said mixture of IL-2A and IL-2B migrates as two bands corresponding to IL-2A and IL-2B, respectively, on SDS-polyacrylamide gel electrophoresis. The mobilities of said mixture of IL-2A and IL-2B under reducing conditions are the same as those under non-reducing conditions. The molecular weights of said mixture of IL-2A and IL-2B are calculated to be 17 ± 1 and 17.5 ± 1 kilodaltons, respectively, based on the results of SDS-polyacrylamide gel electrophoresis.

(2) Isoelectric point

When subjected to the same procedure as that described in Example 2 [2] (3), said mixture of IL-2A and IL-2B is electrophoresed as two bands corresponding to IL-2A and IL-2B. The isoelectric point of the former is 7.2 ± 0.2 and the latter 6.6 ± 0.2.

(3)  Amino acid composition

The amino acid composition of said mixture of IL-2A and IL-2B is determined by the same method as that described in Example 2 [2](4).  The results obtained are shown in Table 7.

### Table 7

| Amino acid | Content (mole %) |
|---|---|
| Asp/Asn | 9.4 ± 2.0 |
| Thr | 9.1 ± 2.6 |
| Ser | 6.8 ± 1.5 |
| Glu/Gln | 14.5 ± 3.0 |
| Gly | 5.1 ± 5.0 |
| Ala | 4.7 ± 1.5 |
| Val | 3.8 ± 1.4 |
| Met | 3.2 ± 0.7 |
| Ile | 6.2 ± 1.8 |
| Leu | 15.1 ± 4.4 |
| Tyr | 2.4 ± 0.5 |
| Phe | 6.2 ± 4.1 |
| Lys | 8.3 ± 1.8 |
| His | 2.0 ± 0.7 |
| Arg | 3.3 ± 0.8 |

The content values given in Table 7 are expressed in terms of mole percent based on the total number of amino acid residues except Pro, 1/2 Cys, and Trp.

(4)  Amino terminal amino acid sequence

The amino terminal amino acid sequence of said mixture of IL-2A and IL-2B is determined by the same method  as that used in Example 2 [2] (5).  The amino terminal amino acid sequence (residues from No. 1 through No. 20) is shown below:

Formula

```
     1   2   3   4   5   6   7   8   9  10  11  12  13  14  15 16
H-Ala-Pro-X-Ser-Ser-Ser-X-Lys-Lys-X-Gln-Leu-Gln-Leu-Glu-X-
```

17  18  19  20
Leu-Leu-Leu-Asp

wherein each X is yet unknown.

(5)  Elution pattern on high performance liquid chromatography

When subjected to reverse phase high performance liquid chromatography under the same conditions as those described in [1], said mixture of IL-2A and IL-2B is resolved into two peaks corresponding to IL-2A-1/IL-2B-1 and IL-2A-2/IL-2B-2.

[3]  Properties of a mixture of IL-2A-1 and IL-2B-1

The mixture of IL-2A-1 and IL-2B-1 obtained in [1] is examined for the following properties:

(1)  Molecular weight

When subjected to the same procedure as that described in Example 2 [2](1), said mixture of IL-2A-1 and IL-2B-1 migrates as two bands corresponding to IL-2A-1 and IL-2B-1 on SDS-polyacrylamide gel electrophoresis.  The mobilities of said mixture of IL-2A-1 and IL-2B-1 under reducing conditions are the same as those under non-reducing conditions. The molecular weights of said mixture of IL-2A-1 and IL-2B-1 are calculated to be $17 \pm 1$ and $17.5 \pm 1$ kilodaltons, respectively, based on the results of SDS-polyacrylamide gel electrophoresis.

(2)  Isoelectric point

When subjected to the same procedure as that described in Example 2 [2](3), said mixture of IL-2A-1 and IL-2B-1 is electrophoresed as two bands corresponding to IL-2A-1 and IL-2B-1.  The isoelectric point of the former is $7.2 \pm 0.2$ and the latter $6.6 \pm 0.2$.

(3)  Amino acid composition

The amino acid composition of said mixture of IL-2A-1 and IL-2B-1 is determined by the same method as that described in Example 2 [2](4).  The results obtained are shown in Table 8.

Table 8

| Amino acid | Content (mole %) |
|---|---|
| Asp/Asn | 9.4 ± 1.9 |
| Thr | 8.8 ± 2.6 |
| Ser | 6.9 ± 1.5 |
| Glu/Gln | 14.6 ± 3.0 |
| Gly | 5.8 ± 5.4 |
| Ala | 5.0 ± 1.5 |
| Val | 4.0 ± 1.4 |
| Met | 3.2 ± 0.7 |
| Ile | 6.1 ± 1.8 |
| Leu | 14.8 ± 4.4 |
| Tyr | 2.5 ± 0.5 |
| Phe | 5.5 ± 1.8 |
| Lys | 8.3 ± 1.8 |
| His | 2.0 ± 0.7 |
| Arg | 3.4 ± 0.8 |

The content values given in Table 8 are expressed in terms of mole percent based on the total number of amino acid residues except Pro, 1/2 Cys, and Trp.

(4) Amino terminal amino acid sequence

The amino terminal amino acid sequence of a mixture of IL-2A-1 and IL-2B-1 is determined by the same method as that used in Example 2 [2] (5). The amino terminal amino acid sequence (residues from No. 1 through No. 14) is shown below:

Formula

```
  1   2   3   4   5   6   7   8   9  10  11  12  13  14
H-Ala-Pro-X-Ser-Ser-Ser-X-Lys-Lys-X-Gln-Leu-Gln-Leu
```

wherein each X is yet unknown.

[4] Properties of a mixture of IL-2A-2 and IL-2B-2

The mixture of IL-2A-2 and IL-2B-2 obtained in [1] is examined for the following properties:

(1) Molecular weight

When subjected to the same procedure as that described in Example 2 [2] (1), said mixture of IL-2A-2 and IL-2B-2 migrates as two bands corresponding to IL-2A-2 and IL-2B-2 on SDS-polyacrylamide gel electrophoresis. The mobilities of said mixture of IL-2A-2 and IL-2B-2 under reducing conditions are the same as those under non-reducing conditions. The molecular weights of said mixture of IL-2A-2 and IL-2B-2 are calculated to be 17 ± 1 and 17.5 ± 1 kilodaltons, respectively, based on the results of SDS-polyacrylamide gel electrophoresis.

(2) Isoelectric point

When subjected to the same procedure as that described in Example 2 [2] (3), said mixture of IL-2A-2, and IL-2B-2 is electrophoresed as two bands corresponding to IL-2A-2 and IL-2B-2. The isoelectric point of the former is 7.2 ± 0.2 and the latter 6.6 ± 0.2.

(3) Amino acid composition

The amino acid composition of said mixture of IL-2A-2 and IL-2B-2 is determined by the same method as that described in Example 2 [2] (4). The results obtained are shown in Table 9.

Table 9

| Amino acid | Content (mole %) |
|---|---|
| Asp/Asn | 9.4 ± 2.0 |
| Thr | 9.3 ± 2.0 |
| Ser | 6.8 ± 1.5 |
| Glu/Gln | 14.5 ± 3.0 |
| Gly | 4.3 ± 4.1 |
| Ala | 4.5 ± 0.9 |
| Val | 3.6 ± 0.7 |
| Met | 3.1 ± 0.7 |
| Ile | 6.3 ± 1.8 |
| Leu | 15.3 ± 4.4 |
| Tyr | 2.4 ± 0.5 |
| Phe | 6.9 ± 4.1 |

| Amino acid | Content (mole %) |
|---|---|
| Lys | 8.4 ± 1.8 |
| His | 2.1 ± 0.6 |
| Arg | 3.2 ± 0.7 |

The content values given in Table 9 are expressed in terms of mole percent based on the total number of amino acid residues except Pro, 1/2 Cys, and Trp.

(4) Amino terminal amino acid sequence

The amino terminal amino acid sequence of said mixture of IL-2A-2 and IL-2B-2 is determined by the same method as that used in Example 2[2] (5). The amino terminal amino acid sequence (residues from No. 1 through No. 20) is shown below:

Formula

```
   1   2   3   4   5   6   7   8   9  10  11  12  13  14  15 16
H-Ala-Pro-X-Ser-Ser-Ser-X-Lys-Lys-X-Gln-Leu-Gln-Leu-Glu-X-
  17  18  19  20
Leu-Leu-Leu-Asp
```

wherein each X is yet unknown.

Concerning the amino acid compositions mentioned in Examples 2 [2](4), 2 [3](4), 2 [4](4), 3 [2](4), 3 [3](4), 3 [4](4), 4 [2](3), 4 [3](3), and 4 [4](3), the presences of Pro, 1/2 Cys, and Trp are established qualitatively based on the results of amino terminal amino acid sequencing, amino acid analysis after performic acid oxidation, and ultraviolet absorption spectrophotometry, respectively.

In Examples 1, 2, 3 and 4, the activity of IL-2 is determined according to the method described in Biochemical and Biophysical Research Communications, 109, 363 (1982).

In Examples 2 [1], 3 [1] and 4 [1], the high performance liquid chromatography is performed using a Varian (USA) model 5040 high performance liquid chromatograph equipped with a Rheodyne (USA) model 7125 injector (loop capacity of 500 μl).

Example 5

Activity against IL-2 dependent cells:

IL-2 activity determination is carried out by the method described in Biochemical and Biophysical Research Communications, 109, 363 (1982). The IL-2A-1, A-2, B-1 and B-2 preparations obtained in Examples 2 and 3 exhibit the activity to promote tritiated thymidine uptake in an IL-2-dependent human cell line (represented by -Δ-),a mouse natural killer cell line (represented by -o-) and a mouse killer T cell line (represented by -o-) as shown in Figs. 4, 5, 6 and 7, respectively.

What we claim is:

1.    Human interleukin 2A which has the following physico-chemical properties:

(i)   Molecular weight:  17 ± 1 kilodaltons (as determined by SDS-polyacrylamide gel electrophoresis),

(ii)  Amino acid composition:  Contains the following amino acids and, in addition, Pro, 1/2 Cys, and Trp:

| Amino acid | Content (mole %) |
|------------|------------------|
| Asp/Asn | 9.1 ± 1.9 |
| Thr | 8.8 ± 2.6 |
| Ser | 7.3 ± 1.5 |
| Glu/Gln | 14.3 ± 3.0 |
| Gly | 5.5 ±.5.4 |
| Ala | 4.9 ± 1.0 |
| Val | 3.6 ± 0.7 |
| Met | 3.2 ± 0.7 |
| Ile | 6.1 ± 1.8 |
| Leu | 14.9 ± 4.4 |
| Tyr | 2.3 ± 0.5 |
| Phe | 6.8 ± 4.1 |
| Lys | 7.9 ± 1.7 |
| His | 2.1 ± 0.4 |
| Arg | 3.3 ± 0.8 |

(The amino acid content values given above are expressed in terms of mole percent based on the total number of amino acid residues except Pro, 1/2 Cys, and Trp.)

(iii) Amino terminal amino acid sequence:  The first and second amino acids from the amino terminal amino acid are Ala-Pro, from the 4th through 6th are Ser-Ser-Ser, 9th is Lys, and from the 11th through 13th are Gln-Leu-Gln, and

(iv)  Isoelectric point: 7.2 ± 0.2 (as determined by isoelectric focusing).

2.    Human interleukin 2B which has the following physico-
chemical properties:

  (i)  Molecular weight:   17.5 ± 1 kilodaltons (as determined by SDS-polyacrylamide gel electrophoresis),

 (ii)  Amino acid composition:  Contains the following
amino acids and, in addition, Pro, 1/2 Cys, and Trp:

| Amino acid | Content (mole %) |
|---|---|
| Asp/Asn | 9.6 ± 2.0 |
| Thr | 9.5 ± 2.0 |
| Ser | 6.4 ± 1.3 |
| Glu/Gln | 14.7 ± 3.0 |
| Gly | 4.1 ± 4.0 |
| Ala | 4.7 ± 1.5 |
| Val | 3.8 ± 1.4 |
| Met | 3.1 ± 0.7 |
| Ile | 6.3 ± 1.8 |
| Leu | 15.4 ± 4.4 |
| Tyr | 2.5 ± 0.5 |
| Phe | 6.0 ± 1.8 |
| Lys | 8.7 ± 1.8 |
| His | 1.9 ± 0.7 |
| Arg | 3.2 ± 0.7 |

(The amino acid content values given above are expressed in terms of mole percent based on the total
number of amino acid residues except Pro, 1/2 Cys,
and Trp.),

(iii)  Amino terminal amino acid sequence:  The first and
second amino acids from the amino terminal amino acid
are Ala-Pro, from the 4th through 6th are Ser-Ser-Ser,
the 8th and 9th are Lys-Lys, from the 11th through
15th are Gln-Leu-Gln-Leu-Glu, and from the 17th through
20th are Leu-Leu-Leu-Asp, and

 (iv)  Isoelectric point: 6.6 ± 0.2 (as determined by
isoelectric focusing).

3.  Human interleukin-2 as claimed in Claim 1, wherein the interleukin-2 has the following physico-chemical properties and named IL-2A-1:

(i) Molecular weight:  17 ± 1 kilodaltons (as determined by SDS-polyacrylamide gel electrophoresis),

(ii) Amino acid composition:  Contains the following amino acids and, in addition, Pro, 1/2 Cys, and Trp:

| Amino acid | Content (mole %) |
|---|---|
| Asp/Asn | 9.2 ± 1.9 |
| Thr | 8.5 ± 2.6 |
| Ser | 7.3 ± 1.5 |
| Glu/Gln | 14.6 ± 3.0 |
| Gly | 6.7 ± 5.4 |
| Ala | 4.9 ± 1.0 |
| Val | 3.5 ± 0.7 |
| Met | 3.5 ± 0.7 |
| Ile | 6.1 ± 1.3 |
| Leu | 15.0 ± 3.0 |
| Tyr | 2.4 ± 0.5 |
| Phe | 4.8 ± 1.0 |
| Lys | 7.8 ± 1.6 |
| His | 2.2 ± 0.5 |
| Arg | 3.6 ± 0.8 |

(The amino acid content values given above are expressed in terms of mole percent based on the total number of amino acid residues except Pro, 1/2 Cys, and Trp.),

(iii) Amino terminal amino acid sequence:  The first and second amino acids from the amino terminal amino acid are Ala-Pro, from the 4th through 6th are Ser-Ser-Ser, and the 11th and 12th are Gln-Leu,

(iv) Isoelectric point:  7.2 ± 0.2 (as determined by isoelectric focusing), and

(v) Retention time on high performance liquid chromatography:  When, following adsorption on an Ultrapore® RPSC column (4.6 x 75 mm), elution is conducted at a
flow rate of 0.8 ml/min. and at 30°C according to
the elution program given below using 0.1% trifluoroacetic acid-99.9% water as solvent A and 0.1% trifluoroacetic acid-99.9% acetonitrile as solvent B, and
IL-2A is resolved into two activity peaks, IL-2A-1
is eluted at a shorter retention time than the other.

| Time (min.) | Solvent A (%) | Solvent B (%) |
|-------------|---------------|---------------|
| 0 | 68 | 32 |
| 25 | 55 | 45 |
| 35 | 45 | 55 |
| 45 | 30 | 70 |
| 48 | 0 | 100 |

4. Human interleukin-2 as claimed in Claim 1, wherein
the interleukin-2 has the following physico-chemical
properties and named IL-2A-2:

  (i) Molecular weight: 17 ± 1 kilodaltons (as determined
     by SDS-polyacrylamide gel electrophoresis),

 (ii) Amino acid composition: Contains the following amino
     acids and, in addition, Pro, 1/2 Cys, and Trp:

| Amino acid | Content (mole %) |
|------------|------------------|
| Asp/Asn | 9.1 ± 1.9 |
| Thr | 8.9 ± 1.8 |
| Ser | 7.2 ± 1.5 |
| Glu/Gln | 14.2 ± 2.9 |
| Gly | 4.9 ± 4.1 |
| Ala | 4.5 ± 0.9 |
| Val | 3.6 ± 0.7 |
| Met | 3.0 ± 0.6 |
| Ile | 6.1 ± 1.8 |
| Leu | 14.8 ± 4.4 |
| Tyr | 2.3 ± 0.5 |
| Phe | 7.8 ± 4.1 |
| Lys | 8.0 ± 1.7 |
| His | 2.1 ± 0.4 |

> Amino acid  Content (mole %)
> Arg     3.2 ± 0.7

>(The amino acid content values given above are expressed in terms of mole percent based on the total number of amino acid residues except Pro, 1/2 Cys, and Trp.),

(iii) Amino terminal amino acid sequence: The first and second amino acids from the amino terminal amino acid are Ala-Pro, from the 4th through 6th are Ser-Ser-Ser, the 9th is Lys, and from 11th through 13th are Gln-Leu-Gln,

(iv) Isoelectric point: 7.2 ± 0.2 (as determined by isoelectric focusing), and

(v) Retention time on high performance liquid chromatography: When, following adsorption on an Ultrapore® RPSC column (4.6 x 75 mm), elution is conducted at a flow rate of 0.8 ml/min. and at 30°C according to the elution program given below using 0.1% trifluoroacetic acid-99.9% water as solvent A and 0.1% trifluoroacetic acid-99.9% acetonitrile as solvent B, and IL-2A is resolved into two activity peaks, IL-2A-2 is eluted at a longer retention time than the other.

| Time (min.) | Solvent A (%) | Solvent B (%) |
| --- | --- | --- |
| 0 | 68 | 32 |
| 25 | 55 | 45 |
| 35 | 45 | 55 |
| 45 | 30 | 70 |
| 48 | 0 | 100 |

5. Human interleukin-2 as claimed in Claim 2, wherein the interleukin-2 has the following physico-chemical properties and named IL-2B-1:

(i) Molecular weight: 17.5 ± 1 kilodaltons (as determined by SDS-polyacrylamide gel electrophoresis),

(ii) Amino acid composition: Contains the following amino
acids and, in addition, Pro, 1/2 Cys, and Trp:

| Amino acid | Content (mole %) |
|---|---|
| Asp/Asn | 9.5 ± 1.9 |
| Thr | 9.1 ± 1.9 |
| Ser | 6.4 ± 1.3 |
| Glu/Gln | 14.6 ± 3.0 |
| Gly | 4.9 ± 4.0 |
| Ala | 5.0 ± 1.5 |
| Val | 4.5 ± 1.4 |
| Met | 2.9 ± 0.6 |
| Ile | 6.0 ± 1.8 |
| Leu | 14.6 ± 4.4 |
| Tyr | 2.5 ± 0.5 |
| Phe | 6.1 ± 1.8 |
| Lys | 8.7 ± 1.8 |
| His | 1.8 ± 0.7 |
| Arg | 3.2 ± 0.7 |

(The amino acid content values given above are expressed
in terms of mole percent based on the total number
of amino acid residues except Pro, 1/2 Cys, and Trp.),

(iii) Amino terminal amino acid sequence: The first and
second amino acids from the amino terminal amino
acid are Ala-Pro, from the 4th through 6th are Ser-
Ser-Ser, the 8th and 9th are Lys-Lys, and from the
11th through 14th are Gln-Leu-Gln-Leu,

(iv) Isoelectric point: 6.6 ± 0.2 (as determined by
isoelectric focusing), and

(v) Retention time on high performance liquid chromatography: When, following adsorption on an Ultrapore®
RPSC column (4.6 x 75 mm), elution is conducted at a
flow rate of 0.8 ml/min. and at 30°C according to
the elution program given below using 0.1% trifluoroacetic acid-99.9% water as solvent A and 0.1% trifluoro-

0132359

- 37 -

acetic acid-99.9% acetonitrile as solvent B, and IL-
2B is resolved into two activity peaks,  IL-2B-1 is
eluted at a shorter retention time than the other.

| Time (min.) | Solvent A (%) | Solvent B (%) |
|:---:|:---:|:---:|
| 0 | 68 | 32 |
| 25 | 55 | 45 |
| 35 | 45 | 55 |
| 45 | 30 | 70 |
| 48 | 0 | 100 |

6.    Human interleukin-2 as claimed in Claim 2, wherein the
interleukin-2 has the following physico-chemical properties
and named IL-2B-2:

   (i) Molecular weight:  17.5 ± 1 kilodaltons (as determined
      by SDS-polyacrylamide gel electrophoresis),

  (ii) Amino acid composition:  Contains the following amino
     acids and, in addition, Pro, 1/2 Cys, and Trp:

| Amino acid | Content (mole %) |
|:---:|:---:|
| Asp/Asn | 9.7 ± 2.0 |
| Thr | 9.7 ± 2.0 |
| Ser | 6.4 ± 1.3 |
| Glu/Gln | 14.7 ± 3.0 |
| Gly | 3.7 ± 3.0 |
| Ala | 4.5 ± 0.9 |
| Val | 3.5 ± 0.7 |
| Met | 3.2 ± 0.7 |
| Ile | 6.4 ± 1.3 |
| Leu | 15.8 ± 3.2 |
| Tyr | 2.5 ± 0.5 |
| Phe | 6.0 ± 1.8 |
| Lys | 8.7 ± 1.8 |
| His | 2.0 ± 0.6 |
| Arg | 3.2 ± 0.7 |

(The amino acid content values given above are expressed
in terms of mole percent based on the total number of
amino acid residues except Pro, 1/2 Cys, and Trp.),

(iii) Amino terminal amino acid sequence: The first and
second amino acids from the amino terminal amino
acid are Ala-Pro, from the 4th through 6th are Ser-Ser
Ser, the 8th and 9th are Lys-Lys, from the 11th
through 15th are Gln-Leu-Gln-Leu-Glu, and from the 17th
through 20th are Leu-Leu-Leu-Asp,

(iv) Isoelectric point: 6.6 ± 0.2 (as determined by
isoelectric focusing), and

(v) Retention time on high performance liquid chromatography: When, following adsorption on an Ultrapore®
RPSC column (4.6 x 75 mm), elution is conducted at a
flow rate of 0.8 ml/min. and at 30°C according to
the elution program given below using 0.1% trifluoroacetic acid-99.9% water as solvent A and 0.1% trifluoroacetic acid-99.9% acetonitrile as solvent B,
and IL-2B is resolved into two activity peaks, IL-
2B-2 is eluted after a longer retention time than
the other.

| Time (min.) | Solvent A (%) | Solvent B (%) |
|---|---|---|
| 0 | 68 | 32 |
| 25 | 55 | 45 |
| 35 | 45 | 55 |
| 45 | 30 | 70 |
| 48 | 0 | 100 |

7. A mixture of human interleukin-2A and human interleukin-2B, which has the physicochemical properties:

(i) Molecular weight: 17 ± 1 kilodaltons and 17.5 ± 1
kilodaltons (as determined by SDS-polyacrylamide gel
electrophoresis),

(ii) Amino acid composition: Contains the following amino
acids and, in addition, Pro, 1/2 Cys, and Trp:

| Amino acid | Content (mole %) |
|------------|------------------|
| Asp/Asn | 9.4 ± 2.0 |
| Thr | 9.1 ± 2.6 |
| Ser | 6.8 ± 1.5 |
| Glu/Gln | 14.5 ± 3.0 |
| Gly | 5.1 ± 5.0 |
| Ala | 4.7 ± 1.5 |
| Val | 3.8 ± 1.4 |
| Met | 3.2 ± 0.7 |
| Ile | 6.2 ± 1.8 |
| Leu | 15.1 ± 4.4 |
| Tyr | 2.4 ± 0.5 |
| Phe | 6.2 ± 4.1 |
| Lys | 8.3 ± 1.8 |
| His | 2.0 ± 0.7 |
| Arg | 3.3 ± 0.8 |

(The amino acid content values given above are expressed in terms of mole percent based on the total number of amino acid residues except Pro, 1/2 Cys, and Trp.),

(iii) Amino terminal amino acid sequence: The first and second amino acids from the N-terminal amino acid are Ala-Pro (1, 2), from the 4th through 6th are Ser-Ser-Ser (4, 5, 6), the 8th and 9th are Lys-Lys (8, 9), from the 11th through 15th are Gln-Leu-Gln-Leu-Glu (11, 12, 13, 14, 15), and from the 17th through 20th are Leu-Leu-Leu-Asp (17, 18, 19, 20), and

(iv) Isoelectric point: 7.2 ± 0.2 and 6.6 ± 0.2 as determined by isoelectric focusing).

8. A mixture of human interleukin 2A-1 and human interleukin-2B-1, which has the physicochemical properties:

(i) Molecular weight: 17 ± 1 kilodaltons and 17.5 ± 1 kilodaltons (as determined by SDS-polyacrylamide gel electrophoresis),

(ii) Amino acid composition:  Contains the following
amino acids and, in addition, Pro, 1/2 Cys, and Trp:

| Amino acid | Content (mole %) |
|---|---|
| Asp/Asn | 9.4 ± 1.9 |
| Thr | 8.8 ± 2.6 |
| Ser | 6.9 ± 1.5 |
| Glu/Gln | 14.6 ± 3.0 |
| Gly | 5.8 ± 5.4 |
| Ala | 5.0 ± 1.5 |
| Val | 4.0 ± 1.4 |
| Met | 3.2 ± 0.7 |
| Ile | 6.1 ± 1.8 |
| Leu | 14.8 ± 4.4 |
| Tyr | 2.5 ± 0.5 |
| Phe | 5.5 ± 1.8 |
| Lys | 8.3 ± 1.8 |
| His | 2.0 ± 0.7 |
| Arg | 3.4 ± 0.8 |

(The amino acid content values given above are expressed
in terms of mole percent based on the total number of
amino acid residues except Pro, 1/2 Cys, and Trp.),

(iii) Amino terminal amino acid sequence: The first and
second amino acids from the N-terminal amino acid
are Ala-Pro, from the 4th through 6th are Ser-Ser-Ser,
the 8th to 9th are Lys-Lys, and from the 11th through
14th are Gln-Leu-Gln-Leu, and

(iv) Isoelectric point:  7.2 ± 0.2 and 6.6 ± 0.2 (as
determined by isoelectric focusing).


9.    A mixture of human interleukin-2A-2 and human interleukin-2B-2 which has the physicochemical properties:

(i) Molecular weight:  17 ± 1 kilodaltons and 17.5 ± 1
kilodaltons (as determined by SDS-polyacrylamide gel electrophoresis);

(ii) Amino acid composition: Contains the follwoing amino
acids and, in addition, Pro, 1/2 Cys, and Trp:

| Amino acid | Content (mole %) |
|------------|------------------|
| Asp/Asn | 9.4 ± 2.0 |
| Thr | 9.3 ± 2.0 |
| Ser | 6.8 ± 1.5 |
| Glu/Gln | 14.5 ± 3.0 |
| Gly | 4.3 ± 4.1 |
| Ala | 4.5 ± 0.9 |
| Val | 3.6 ± 0.7 |
| Met | 3.1 ± 0.7 |
| Ile | 6.3 ± 1.8 |
| Leu | 15.3 ± 4.4 |
| Tyr | 2.4 ± 0.5 |
| Phe | 6.9 ± 4.1 |
| Lys | 8.4 ± 1.8 |
| His | 2.1 ± 0.6 |
| Arg | 3.2 ± 0.7 |

(The amino acid content values given above are expressed
in terms of mole percent based on the total number of
amino acid residues except Pro, 1/2 Cys, and Trp.)

(iii) Amino terminal amino acid sequence: The first and second
amino acids from the N-terminal amino acid are

$$\overset{1}{Ala}-\overset{2}{Pro}, \text{ from the 4th through 7th are } \overset{4}{Ser}-\overset{5}{Ser}-\overset{6}{Ser},$$

$$\text{the 8th to 9th are } \overset{8}{Lys}-\overset{9}{Lys}, \text{ from the 11th through 15th are}$$

$$\overset{11}{Gln}-\overset{12}{Leu}-\overset{13}{Gln}-\overset{14}{Leu}-\overset{15}{Glu}, \text{ and from the 17th through 20th are}$$

$$\overset{17}{Leu}-\overset{18}{Leu}-\overset{19}{Leu}-\overset{20}{Asp},$$

(iv) Isoelectric point: 7.2 ± 0.2 and 6.6 ± 0.2 (as determined by isoelectiric focusing)

10. A method of producing human IL-2A, B or a mixture thereof ,which
comprises cultivating human cells capable of producing IL-2 in the presence of an inducer and thereby causing formation and accumulation of human IL-2A and B in the culture broth and subjecting the culture fluid to a series of steps, namely the steps of:

(a) applying the culture fluid to a sulfopropyl-crosslinked dextran column, for adsorption, and performing elution with a buffer,

(b) applying the eluate to a column packed with an anion exchange chromatographic carrier, for adsorption, and conducting elution with a salt solution,

(c) subjecting the eluate to gel filtration on a polyacrylamide-agarose column,

(d) applying the eluate to a DEAE-cellulose column, for adsorption, and carrying out elution of activity peaks with salt solutions, the activity peak being eluted at a lower salt concentration to give IL-2A-containing solution and at a higher salt concentration to give IL-2B-containing solution,

and then, when the IL-2A-containing solution is subjected to the following steps, IL-2A is obtained; when the IL-2B-containing solution is subjected to the following steps, IL-2B is obtained; and when both the IL-2A-containing solution and the IL-2-containing solution are subjected to the following steps, a mixture of IL-2A and B is obtained:

(e) subjecting the eluate to the second gel filtration on a polyacrylamide-agarose column, and

(f) subjecting the eluate to high performance liquid chromatography and performing elution with a solvent.

11. The method of Claim 10, wherein the human cells capable of producing IL-2 are peripheral blood-derived lymphocytes.

12. The method of Claim 10, wherein the cultivation is carried out in a serum-free medium.

13.    The method of Claim 10, wherein the inducer is
an alloantigen, TPA, Con A or a mixture thereof.

14.    A method of producing human IL-2 A-1 or A-2, which
comprises recovering the activity peaks of human IL-2A
obtained in Claim 10, the activity peak being eluted at
a shorter retention time than the other on high performance
liquid chromatography, to give human IL-2A-1; or comprises
recovering the activity peaks of human IL-2A obtained
in Claim 10, the activity peak being eluted at a longer
retention time than the other on high performance liquid
chromatography, to give human IL-2 A-2.

15.    A method of producing human IL-2 B-1 or B-2, which
comprises recovering the activity peaks of human IL-2B
obtained in Claim 10, the activity peak being eluted at
a shorter retention time than the other on high performance
liquid chromatography, to give human IL-2B-1; or comprises
recovering the activity peaks of human IL-2B obtained in
Claim 10, the activity peak being eluted at a longer
retention time than the other on high performance liquid
chromatography, to give human IL-2 B-2.

16.    A method of producing a mixture of human IL-2A and B,
which comprises cultivating human cells capable of producing
IL-2 in the presence of an inducer and thereby causing formation
and accumulation of human IL-2A and B in the culture broth
and subjecting the culture fluid to a series of steps,
namely the steps of:
(a)    applying the culture fluid to a sulfopropyl-crosslinked dextran column, for adsorption, and performing
       elution with a buffer,
(b)    applying the eluate to a column packed with an
       anion exchange chromatographic carrier, for adsorption,
       and conducting elution with a salt solution,

(c)     subjecting the eluate to gel filtration on a polyacrylamide-agarose column, and

(d)     sujecting the eluate to high performance liquid
        chromatography and performing elution with a solvent.

17.     The method of Claim 16, wherein the human cells
capable of producing IL-2 are peripheral blood-derived
lymphocytes.

18.     The method of Claim 11, wherein the cultivation is
carried out in a serum-free medium.

19.     The method of Claim 16, wherein the inducer is
an alloantigen, TPA, Con A or a mixture thereof.

20.     A method of producing a mixture of human IL-2A-1
and B-1, which comprises recovering the activity peak of
human IL-2A and B obtained in claim 16, the activity peak
being eluted at a shorter retention time than the other
on high performance liquid chromatography.

21.     A method of producing a mixture of human IL-2A-2
and B-2, which comprises recovering the activity peak
of human IL-2A and B obtained in Claim 16, the activity peak
being eluted at a longer retention time than the other on
high performance liquid chromatography.

Fig. 1

Fig. 2

0132359
1/4

A A-1 A-2 B-1 B-2 B

Standard proteins   Purified human IL-2   Standard proteins

**Fig.3**

A-1

Tritiated thymidine uptake ( ×10⁻⁴ c.p.m.)

Dilution factor (log₂)

**Fig.4**

Fig.5

Fig.6

Fig.7